# EUROPÄISCHE PATENTSCHRIFT

(11) **EP 1 394 269 B1**
(45) Veröffentlichungstag und Bekanntmachung des Hinweises auf die Patenterteilung: **02.03.2005**
(21) Anmeldenummer: 03018362.8
(22) Anmeldetag: 13.08.2003
(51) Int. Cl.: C12Q 1/68

(54) **Verfahren zur Isolierung biologischer Makromoleküle sowie Vorrichtung zur Durchführung dieses Verfahrens**
Method for the isolation of biological macromolecules and apparatus for conducting said method
Procédé et dispositif pour l'isolation de macromolécules biologiques

(30) Priorität: 19.08.2002 DE 10238630
(43) Veröffentlichungstag der Anmeldung: 03.03.2004
(73) Patentinhaber: Macherey, Nagel GmbH & Co. Handelsgesellschaft, 52355 Düren (DE)
(72) Erfinder: Zinn, Thomas, Dr., 52355 Düren (DE); Möller, Klaus, Dr., 52249 Eschweiler (DE); Radmacher, Edmund, Dr., 52349 Düren (DE)
(74) Vertreter: Paul, Dieter-Alfred, Dipl.-Ing.

(56) Entgegenhaltungen:
- EP-A- 1 125 943
- WO-A-00/05358
- WO-A-01/79486
- WO-A-97/29113
- US-B1- 6 395 231

## Beschreibung

Die Erfindung betrifft ein Verfahren zur Isolierung biologischer Makromoleküle, insbesondere von Plasmid-DNA, aus biologischem Ausgangsmaterial, bei dem aus dem Ausgangsmaterial durch Aufschluß ein Lysat gebildet, dies gereinigt und die so gewonnene Lösung auf ein chromatographisches Material gegeben wird, das die Makromoleküle adsorbiert, wobei zuvor oder gleichzeitig eine Abreicherung von Endotoxinen durchgeführt wird, und bei dem die aufgereinigten Makromoleküle mit Hilfe eines Elutionsmittels von dem chromatographischen Material eluiert und von dem Elutionsmittel befreit werden. Die Erfindung betrifft des weiteren eine Vorrichtung zur Durchführung dieses Verfahrens.

Zur Aufreinigung und Isolierung biologischer Makromoleküle wie Plasmid-DNA oder rekombinant hergestellter Proteine wird biologisches Ausgangsmaterial wie Bakterien, Hefen oder Zellkulturen verwendet, das die biologischen Makromoleküle enthält. Die Zellen werden zunächst aufgeschlossen, beispielsweise mittels alkalischer Lyse. Das so gewonnene Lysat wird dann zwecks Entfernung der Zelltrümmer und anderer Verunreinigungen vorgereinigt, beispielsweise mittels Filtrierung, Zentrifugierung und/oder Fixierung an einem chromatographischen Material. Die so gewonnene Lösung (cleared lysat) wird - gegebenenfalls nach weiteren Behandlungsschritten - auf ein chromatographisches Material gegeben, das geeignet ist, die Makromoleküle aus der Lösung zu adsorbieren. Nach entsprechenden Waschschritten zur Entfernung unerwünschter Begleitstoffe werden die Makromoleküle mittels eines Elutionsmittels, enthaltend eine hohe Konzentration chaotroper Salze, von dem chromatographischen Material eluiert, auf einer Silicamembran adsorbiert, mit ethanolhaltigem Waschpuffer gewaschen und mit Wasser eluiert (vgl. WO 93/11221).

Nachteilig bei diesem Verfahren ist die Verwendung gesundheitsgefährdender Salze und ethanolischer Waschpuffer zur Salzentfernung. Erfolgt die Entfernung des Ethanols vor dem Elutionsschritt nicht vollständig, können insbesondere nachfolgende enzymatische Reaktionen durch Ethanolreste inhibiert werden. Außerdem kommt es häufig zu Ablösungen von Silicafasern aus der Silicamembran, die Folgeanwendungen, wie zum Beispiel PCR und Transfektionsstudien, stören.

Gram-negative Bakterien, wie E. coli, enthalten als Zellbestandteil häufig einen physiologisch bedenklichen Gehalt an Lipopolysacchariden (Endotoxine). Sie werden durch das vorbeschriebene Verfahren allein nicht ausreichend entfernt. Deshalb sind zur Abreicherung der Endotoxine zusätzliche Verfahrensschritte entwickelt worden, beispielsweise Ultrafiltration, Adsorption an Aktivkohle, Ionenaustauscherchromatographie oder Affinitätschromatographie (vgl. S. K. Sharma, "Endotoxin Detection and Elimination in Biotechnology", Biotechnology and Applied Biochemistry 8, 1986, Seite 5 ff). In der US-A-4,412,985 und der WO 95/21179 wird vorgeschlagen, das nach dem Zellaufschluß gewonnene Lysat mit einer wässrigen Salzlösung und Detergenzien, wie beispielsweise Triton® X-114 oder X-100, vorzuinkubieren und dann mit einem Ionenaustauschermaterial zu behandeln.

Bei dem Verfahren nach der WO 99/63076 wird die Lösung auf eine chromatographische Säule gegeben, die sowohl die Plasmid-DNA als auch die Endotoxine adsorbiert. Dann werden die Endotoxine mittels Alkohol oder Essigsäure ausgewaschen. Zusätzlich kann vor der Aufbringung der Lösung auf die Chromatographiesäule ein nicht-ionisches Tensid, beispielsweise Triton® X-114, in die Lösung gegeben werden, wobei die Tensidphase vor der Aufbringung der Lösung auf die Chromatographiesäule entfernt wird. In einem weiteren Schritt wird dann die Plasmid-DNA eluiert, mit Ethanol präzipitiert und einer Sterilfiltration unterworfen.

Eine Weiterentwicklung des Aufreinigungsverfahrens ist in der EP-A-1 125 943 beschrieben. Zwecks Abreicherung der Endotoxine wird hier ein nicht-ionisches Tensid in die Lösung unmittelbar vor oder während der Aufreinigung der Makromoleküle am chromatographischen Material gegeben.

Hierdurch wird das Aufreinigungsverfahren wesentlich beschleunigt.

In der WO 00/05358 ist ein weiteres Verfahren zur Gewinnung endotoxinarmer Plasmid-DNA beschrieben. Bei diesem Verfahren wird die Lösung vor der Aufbringung auf einen Anionenaustauscher einer tangentialen Ultrafiltration unterworfen. Nach der Aufbringung auf den Anionenaustauscher werden die schwach gebundenen Verunreinigungen, beispielsweise Proteine und RNA, und danach die Plasmid-DNA eluiert. An die Eluierung kann sich eine Filtrierung und eine weitere Aufkonzentrierung durch Diafiltrierung mittels tangentialer Ultrafiltration und dann eine zusätzliche Filtrierung zur Sterilisierung anschließen. Dieses Verfahren erfordert wegen vieler Filtrationsschritte einen relativ großen apparativen und kostenverursachenden Aufwand und läßt sich, wenn überhaupt, nur mit hohem Aufwand parallelisieren und automatisieren.

Einen anderen Weg zur Abreicherung von Endotoxinen beschreibt die WO 97/29113. Bei diesem Verfahren wird die Lösung an einem Ionenaustauscher und an Hydroxylapatit chromatographiert. Anschließend wird die DNA eluiert und eine Isopropanol-Ethanol-Fällung durchgeführt. Das Verfahren ist nicht zuletzt wegen eines weiteren Chromatographieschrittes kompliziert und deshalb nicht automatisierbar.

In der WO 01/79486 ist ein Verfahren zur Gewinnung von Plasmid-DNA beschrieben, bei dem die Aufreinigung und Isolierung der DNA über mehrere Filter, und zwar Mikrofilter und Ultrafiltrationsmembranen, erfolgt. Dabei wird auch vorgeschlagen, die Filtrierung durch Über- und Unterdruckaufprägung zu unterstützen. Hierzu werden in einem druckdichten Gehäuse untereinander zwei Mikrotiterplatten mit beispielsweise 96 durchgehenden zylindrischen Kavitäten angeordnet, wobei die Kavitäten der oberen und der unteren Mikrotiterplatten jeweils koaxial übereinander liegen. Nach dem Filtrierungsvorgang wird die Plasmid-DNA von den Oberflächen der Ultrafiltrationsmembranen in der unteren Platte als Retentat abgeschöpft, beispielsweise mittels Pipettieren. Mit einer solchen Filtration lassen sich allerdings Endotoxine nicht auf ein Maß abreichern, das eine Verwendung der gewonnenen Nukleinsäure in weitergehenden Verfahren, z.B. Transfektionsstudien, erlaubt.

Im übrigen offenbaren die DE-C-41 43 394, die US-A-5,283,039 und die DE-U-297 09 916.7 Vakuumvorrichtungen, mit denen Flüssigkeitsproben separiert werden können. Hierzu werden Mikrotiterplatten - auch in Streifenform - verwendet, in deren Kavitäten Filter angeordnet sind. Durch Vakuumbeaufschlagung wird die zu trennende Flüssigkeit durch die Filter gesogen und das Filtrat in Aufnahmeröhrchen aufgefangen.

Der Erfindung liegt die Aufgabe zugrunde, ein robustes und einfach automatisierbares Verfahren zur Aufreinigung biologischer Makromoleküle bereitzustellen, das hohe Ausbeuten für die Zielmoleküle ermöglicht und die Gewinnung der gereinigten Makromoleküle in sehr kleinem Volumen und einer hohen Konzentration sowie frei von partikulären Verunreinigung erlaubt.

Diese Aufgabe wird erfindungsgemäß durch folgende Verfahrensschritte gelöst:
(1) in einer Vorrichtung werden eine erste Mikrotiterplatte mit mehreren parallelen ersten Kavitäten und eine zweite Mikrotiterplatte mit mehreren zweiten Kavitäten derart angeordnet, daß jeweils eine erste und eine zweite Kavität hintereinander liegen;
(2) in den ersten Kavitäten wurden Filtereinheiten angeordnet;
(3) in den zweiten Kavitäten wurde das chromatographische Material angeordnet;
(4) in die ersten Kavitäten wird das Lysat eingegeben;
(5) die aus den ersten Kavitäten austretende Lösung wird direkt in die zweiten Kavitäten überführt, und die Makromoleküle werden am chromatographischen Material adsorbiert;
(6) Endotoxine und weitere Verunreinigungen werden mit einem Waschmittel ausgewaschen;
(7) der zweiten Mikrotiterplatte wird eine dritte Mikrotiterplatte mit mehreren parallelen dritten Kavitäten so zugeordnet, daß jeweils eine zweite und eine dritte Kavität axial hintereinander ausgerichtet sind;
(8) in den dritten Kavitäten wurden Ultrafiltrationseinheiten angeordnet;
(9) in die zweiten Kavitäten wird das Elutionsmittel aufgegeben, und eine Mischung aus Elutionsmittel und Makromolekülen wird in die dritten Kavitäten überführt;
(10) die dritten Kavitäten werden einer in Richtung deren Austrittsseite Kraft ausübenden Druckdifferenz und/oder Fliehkraft ausgesetzt;
(11) danach werden die Makromoleküle als Retentat gewonnenen.

Grundgedanke der Erfindung ist es also, das aus dem Aufschluß der biologischen Ausgangsmaterialien gewonnene Lysat mit Hilfe von Mikrotiterplatten - sie können auch in Streifenform vorliegen - einer ersten Filtration, beispielsweise einer Mikrofiltration, einer anschließenden Adsorption der gewünschten Makromoleküle an einem chromatographischen Material unter Abreicherung der Endotoxine mit Hilfe eines Waschmittels und das Eluat mit den Makromolekülen unmittelbar anschließend einer Ultrafiltration zu unterwerfen. Dabei wird der Ultrafiltrationsschritt durch Einwirken eines Vakuums auf die Ausgangsseite bzw. eines Überdrucks auf die Eingangsseite der dritten Mikrotiterplatte oder durch Einwirkung von Fliehkraft unterstützt. Die Verwendung von Mikrotiterplatten erlaubt die Durchführung des Verfahrens auf gängigen Pipettierrobotern, so daß das Verfahren vollautomatisch und im Hochdurchsatzmaßstab durchgeführt werden kann. Durch den Ultrafiltrationsschritt im Anschluß an den chromatographischen Aufreinigungsschritt kann die Salzentfernung ohne einen zeitaufwendigen Fällungsschritt mit Alkohol durchgeführt werden. Eine Inhibierung nachfolgender enzymatischer Reaktionen durch Ethanolreste ist ausgeschlossen. Die aufgereinigten Makromoleküle werden in hoher Ausbeute und hochreiner Form, sowie in hoher Konzentration bei kleinem Volumen (< 75 µl) erhalten. Damit wird der direkte Einsatz der isolierten Makromoleküle ohne weitere Aufkonzentrierungsschritte oder Nachreinigungsschritte für Folgeapplikationen, z.B. Transfektionsstudien, möglich.

Zur Beschleunigung des Filtrationsvorgangs in den Filtereinheiten der ersten Mikrotiterplatte ist es zweckmäßig, daß nach Aufgabe des Lysats in die ersten Kavitäten diesen eine in Richtung auf die zweiten Kavitäten kraftausübende Druckdifferenz und/oder Fliehkraft aufgeprägt wird, d.h. bei beiden Filtrationsschritten wird die Filtration durch Aufprägen eines Vakuums, eines Überdrucks und/oder einer Fliehkraft unterstützt.

In manchen Fällen ist der Durchflußwiderstand in der zweiten Mikrotiterplatte so gering, daß die von der ersten Mikrotiterplatte kommende Lösung das Chromatographiematerial nur aufgrund von Schwerkrafteinwirkung durchläuft und daß anschließend auch die Elution der Makromoleküle lediglich unter Schwerkrafteinwirkung stattfinden kann. Behindert oder verhindert der Durchflußwiderstand in der zweiten Mikrotiterplatte den Durchfluß der Lösung und des Elutionsmittels, sollten auch die zweiten Kavitäten jeweils eine in Richtung deren Austrittsseite Kraft ausübenden Druckdifferenz und/oder Fliehkraft ausgesetzt werden. Die dritte Mikrotiterplatte fängt dabei das Eluat auf und wird anschließend ebenfalls einer in Richtung der Austrittsseite wirkenden Druckdifferenz oder Fliehkraft ausgesetzt.

Um für das beschriebene Verfahren gebräuchliche Vakuum- oder Überdruckvorrichtungen verwenden zu können, sollten nach Überführung der Lösung in die zweiten Kavitäten die erste Mikrotiterplatte gegen die zweite Mikrotiterplatte ausgetauscht werden, so daß letztere in die Position der ersten Mikrotiterplatte kommt und dann wie bei dem ersten Filtrationsvorgang einer Druckdifferenz und/oder einer Fliehkraft ausgesetzt wird.

Entsprechendes kann für den Elutionsvorgang vorgesehen werden, in dem die erste Mikrotiterplatte gegen die zweite Mikrotiterplatte ausgetauscht wird - wenn dies nicht schon für den Durchfluß der Lösung geschehen ist - und die dritte Mikrotiterplatte in die vorherige Position der zweiten Mikrotiterplatte gebracht wird. Hierdurch ergeben sich konstruktiv einfache Ausführungen für die Vorrichtung, da lediglich erste und zweite Stützflächen zur Abstützung der Mikrotiterplatten vorhanden sein müssen.

Als waschwirksame Substanzen zur Entfernung der Endotoxine haben sich nicht-ionische Tenside, beispielsweise Triton® X-114, in einer Konzentration von 0,1 bis 10 % bewährt. Dabei kann das Waschmittel als Bestandteil von Waschlösungen vorliegen und/oder an Einsätze, beispielsweise Fritten, gebunden sein, die in die zweiten Kavitäten eingebracht wurden. Die Fritten können aus Glas oder Kunststoff bzw. aus verdichteten oder miteinander verwobenen synthetischen Vliesen aus synthetischen Polymermaterialien oder Zellstoffen bestehen. Anstatt oder in Kombination dazu kann auch das chromatographische Material mit dem Waschmittel imprägniert sein.

Als chromatographisches Material für die Isolierung von DNA ist vorzugsweise ein Anionenaustauschermaterial vorgesehen. Es kann aus einem Partikelbett, einem porösen monolithischen Block oder einer chromatographischen Trennmembran bestehen.

Es versteht sich, daß das chromatographische Material vor der Eluierung der Makromoleküle mehrfach gewaschen werden kann, um die Endotoxine und andere Verunreinigungen - beispielsweise bei der Isolierung von DNA Proteine und RNA - zu entfernen.

Die Filtereinheiten können als Mikrofilter ausgebildet sein und mehrstufig wirken, wobei die Feinheit der Filter in Durchströmrichtung zunimmt.

Für die Trennung des Elutionsmittels von den Makromolekülen können auch mehrere dritte Mikrotiterplatten verwendet werden, um eine mehrstufige Ultrafiltration durchzuführen.

Die Vorrichtung zur Durchführung des vorbeschriebenen Verfahrens weist erfindungsgemäß folgende Merkmale auf:
(12) die Vorrichtung hat ein Gehäuse;
(13) die Vorrichtung weist erste, zweite und dritte Mikrotiterplatten mit jeweils mehreren parallelen ersten, zweiten bzw. dritten Kavitäten in übereinstimmendem Raster auf;
(14) in den ersten Kavitäten sind Filtereinheiten angeordnet;
(15) in den zweiten Kavitäten ist chromatographisches Material angeordnet;
(16) in den dritten Kavitäten sind Ultrafiltrationseinheiten angeordnet;
(17) die Vorrichtung weist eine erste Stützfläche für eine randseitig abdichtende Auflage der Mikrotiterplatten auf;
(18) die Vorrichtung weist eine zweite Stützfläche für die Mikrotiterplatten auf;
(19) die Stützflächen sind so angeordnet, daß die Kavitäten von zwei darauf aufgelegten Mikrotiterplatten jeweils axial hintereinander liegen;
(20) der auf der ersten Stützfläche aufliegenden Mikrotiterplatte ist ein Differenzdruck aufprägbar, der in zumindest einer Kavität dieser Mikrotiterplatte eine in Richtung auf die zweite Stützfläche wirkende Kraft ausübt.

Zur Verwirklichung des Verfahrens wird also eine pneumatische Vorrichtung verwendet, bei der die Filtrierungsvorgänge durch Über- oder Unterdruckaufprägung unterstützt werden. Dabei kommen zumindest, aber auch vorzugsweise drei Mikrotiterplatten zur Anwendung, von denen die erste mit Filtereinheiten, insbesondere Mikrofiltereinheiten, die zweite mit chromatographischem Material und die dritte mit Ultrafiltrationseinheiten versehen sind.

In Ausgestaltung dieser Vorrichtung ist vorgesehen, daß das Gehäuse ein Unterteil mit Auffangwanne und ein darauf abnehmbar aufgesetztes Oberteil aufweist und daß die zweite Stützfläche am Unterteil so angeordnet ist, daß eine darauf aufliegende Mikrotiterplatte nach Entfernung des Oberteils herausnehmbar ist. Die erste Stützfläche sollte am Oberteil so angeordnet sein, daß die darauf angeordnete erste Mikrotiterplatte gegen die zweite Mikrotiterplatte und diese gegen die dritte Mikrotiterplatte austauschbar sind.

Nach einem weiteren Merkmal der Erfindung ist vorgesehen, daß der von Ober- und Unterteil umschlossene Raum mit einer Unterdruckquelle verbunden ist. Dies erleichtert die Handhabung der Mikrotiterplatten. Alternativ oder in Kombination dazu kann vorgesehen sein, daß ein Raum mit zumindest einer Kavität, vorzugsweise allen Kavitäten, der auf der ersten Stützfläche ruhenden Mikrotiterplatte auf der Seite dieser Mikrotiterplatte verbunden ist, der der zweiten Stützfläche abgewandt ist, und daß dieser Raum an eine Überdruckquelle angeschlossen ist.

Die Filtereinheiten in der ersten Mikrotiterplatte können mehrschichtig ausgebildet sein, wobei die Feinheit des Filters in Durchströmrichtung zunimmt. Beispielsweise können wenigstens ein Grob- und wenigstens ein Feinfilter vorgesehen sein. Für die Gewinnung von DNA sollte das chromatographische Material ein Anionenaustauschermaterial sein. Dabei kann das chromatographische Material zwischen zwei Fritten angeordnet sein, wobei die Fritten mit einem die Abreicherung von Endotoxinen bewirkenden Waschmittels versehen, insbesondere imprägniert sein können. Alternativ oder in Kombination dazu kann auch das chromatographische Material mit einem die Abreicherung von Endotöxinen bewirkenden Waschmittel, beispielsweise einem nicht-ionischen Tensid, versehen sein.

In der Zeichnung ist die Erfindung anhand eines Ausführungsbeispiels näher veranschaulicht. Es zeigen:
- Figur 1: einen Vertikalschnitt durch ein Vakuumgerät mit der ersten und zweiten Mikrotiterplatte;
- Figur 2: das Vakuumgerät gemäß Figur 1 im Vertikalschnitt mit der zweiten und dritten Mikrotiterplatte;
- Figur 3: einen Vertikalschnitt durch ein Überdruckgerät mit der ersten und zweiten Mikrotiterplatte und
- Figur 4: eine Variante des Überdruckgeräts gemäß Figur 3 im Vertikalschnitt mit der ersten und zweiten Mikrotiterplatte.

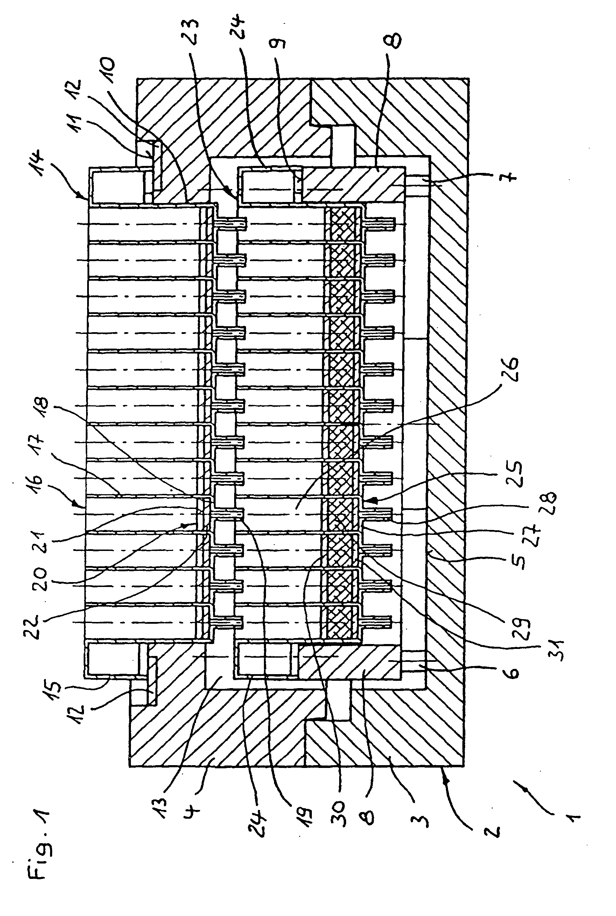

Das in Figur 1 dargestellte Vakuumgerät 1 weist ein Gehäuse 2 rechteckigen Grundrisses auf, das aus einem wannenförmigen Unterteil 3 und einem darauf aufgesetzten Oberteil 4 besteht. Das Unterteil 3 hat einen Boden 5, auf dem über Stützen 6, 7 ein rechteckiger Abstützrahmen 8 mit einer obenseitigen Stützfläche 9 aufsteht. Das Oberteil 4 hat eine rechteckige Öffnung 10, die von einer Stützfläche 11 umgeben ist, welche mit einer Flachdichtung 12 aus einem elastomeren Material versehen ist. Unterteil 3 und Oberteil 4 liegen über eine hier nicht näher dargestellte Dichtung gasdicht aufeinander. Der von ihnen eingeschlossene Innenraum 13 kann über einen hier nicht näher dargestellten Kanal im Unterteil 3 mit einer Vakuumpumpe verbunden werden.

In die Öffnung 10 ist eine erste Mikrotiterplatte 14 eingesetzt. Die Mikrotiterplatte 14 hat einen im Querschnitt L-förmigen Randsteg 15, der mit seiner unteren Stirnseite abdichtend auf der Flachdichtung 12 aufliegt. Die Öffnung 10 und die Mikrotiterplatte 14 sind so aneinander angepaßt, daß zwischen ihnen nur ein geringer Abstand vorhanden ist. Die erste Mikrotiterplatte 14 hat insgesamt sechsundneunzig erste Kavitäten 16, von denen hier nur eine Reihe mit zwölf ersten Kavitäten 16 zu sehen ist. Senkrecht zur Zeichnungsebene sind nebeneinander acht Reihen von solchen Kavitäten 16 angeordnet, so daß die erste Mikrotiterplatte 14 ein Raster von 12x8 Kavitäten 16 hat.

Die ersten Kavitäten 16 haben jeweils einen zylindrischen Abschnitt - beispielhaft mit 17 bezeichnet -, der untenseitig in einem ringförmigen Absatz - beispielhaft mit 18 bezeichnet - endet, der den Querschnitt auf eine Auslauftülle - beispielhaft mit 19 bezeichnet - verengt. Auf den Absätzen 18 ruhen jeweils Filtereinheiten - beispielhaft mit 20 bezeichnet -, die aus einem oberen Grobfilter - beispielhaft mit 21 bezeichnet - und einem unteren Feinfilter - beispielhaft mit 22 bezeichnet - bestehen.

Auf dem Abstützrahmen 8 ruht eine zweite Mikrotiterplatte 23, die gleich der ersten Mikrotiterplatte 14 ausgebildet ist. Sie hat einen umlaufenden Randsteg 24, der auf der Stützfläche 9 aufsteht. Die zweite Mikrotiterplatte 23 hat ebenfalls 96 zweite Kavitäten - beispielhaft mit 25 bezeichnet - in 12x8-Raster. Die zweiten Kavitäten 25 bestehen aus einem oberen zylindrischen Abschnitt - beispielhaft mit 26 bezeichnet -, der untenseitig in einem ringförmigen Absatz - beispielhaft mit 27 bezeichnet - endet, von dem sich nach unten eine Auslauftülle - beispielhaft mit 28 bezeichnet - anschließt.

Der einzige Unterschied zur ersten Mikrotiterplatte 14 besteht darin, daß die zweiten Kavitäten 25 der zweiten Mikrotiterplatte 23 mit Chromatographiematerial - beispielhaft mit 29 bezeichnet - versehen sind. Das Chromatographiematerial 29 ist mit einem geeigneten Puffer äquilibriert und ist beidseitig von Fritten - beispielhaft mit 30 bzw. 31 bezeichnet - eingeschlossen, wobei die jeweils unteren Fritten 31 auf den Absätzen 27 ruhen. Die oberen Fritten 30 sind mit einem nicht-ionischen Tensid, beispielsweise Triton® X-114, imprägniert. Auch das Chromatographiematerial 29 kann mit einem solchen Tensid versehen sein.

Die Stützflächen 9, 11 sind so angeordnet, daß jeweils eine zweite Kavität 25 koaxial unterhalb einer ersten Kavität 16 liegt. Dabei ragen die Auslauftüllen 28 der ersten Kavitäten 16 in die Abschnitte 26 der zweiten Kavitäten 25 hinein.

Zur Isolierung biologischer Makromoleküle wird Zellmaterial zunächst unter Bildung eines Lysats aufgeschlossen wie dies im Stand der Technik bekannt ist. Das Lysat wird dann in die ersten Kavitäten 16 einpipettiert, vorzugsweise unter Verwendung eines Pipettierroboters. Danach wird der Innenraum 13 des Vakuumgerätes 1 durch Anschalten der Vakuumpumpe unter Vakuum gesetzt. Hierdurch wird das Lysat durch die Filtereinheiten 20 gesaugt und strömt dann über die Auslauftüllen 19 in die zweiten Kavitäten 25 der zweiten Mikrotiterplatte 23 als Lösung, d.h. als geklärtes Lysat ein.

Sofern das Chromatographiematerial 29 und die Fritten 30, 31 relativ durchlässig sind (erste Variante), durchströmt die Lösung die oberen Fritten 30, das Chromatographiematerial 29 und die unteren Fritten 31. Dabei adsorbieren die zu gewinnenden Makromoleküle, beispielsweise Plasmid-DNA oder Proteine, am Chromatographiematerial 29, während die meisten anderen Bestandteile der Lösung über die Auslauftüllen 28 nach unten ausströmen. Dabei werden aus den oberen Fritten 30 und dem puffergetränkten Chromatographiematerial 29 nicht-ionische Tenside gelöst, die das Auswaschen der Endotoxine bewirken. Nach Entfernung der ersten Mikrotiterplatte 14 werden Waschpuffer, eventuell mit Tensiden, in die Kavitäten 25 der zweiten Mikrotiterplatte 23 eingegeben. Sie dringen aufgrund Schwerkrafteinwirkung in das Chromatographiematerial 29 ein und unterstützen die Aufreinigung der zu gewinnenden Makromoleküle.

Sofern der Durchlauf der Lösung und des Waschpuffers durch das Chromatographiematerial 29 und die Fritten 30 (zweite Variante), 31 sehr behindert oder sogar verhindert ist, wird die erste Mikrotiterplatte 14 nach Sammeln der Lösung in den zweiten Kavitäten 25 von dem Oberteil 4 abgenommen und das Oberteil 4 von dem Unterteil 3 abgehoben. Dann wird die zweite Mikrotiterplatte 23 entnommen, das Oberteil 4 wieder auf das Unterteil 3 aufgesetzt und die zweite Mikrotiterplatte 23 in die Öffnung 10 des Oberteils 4 eingesetzt, so daß sie dann die ehemalige Position der ersten Mikrotiterplatte 14 einnimmt. Danach wird der Innenraum 13 durch Anschalten der Vakuumpumpe wieder unter Vakuum gesetzt mit der Folge, daß die Lösung das Chromatographiematerial 29 unter Adsorbtion der zu gewinnenden Makromoleküle durchläuft. Für die anschließende Aufgabe des Waschpuffers wird die Vakuumbeaufschlagung fortgesetzt.

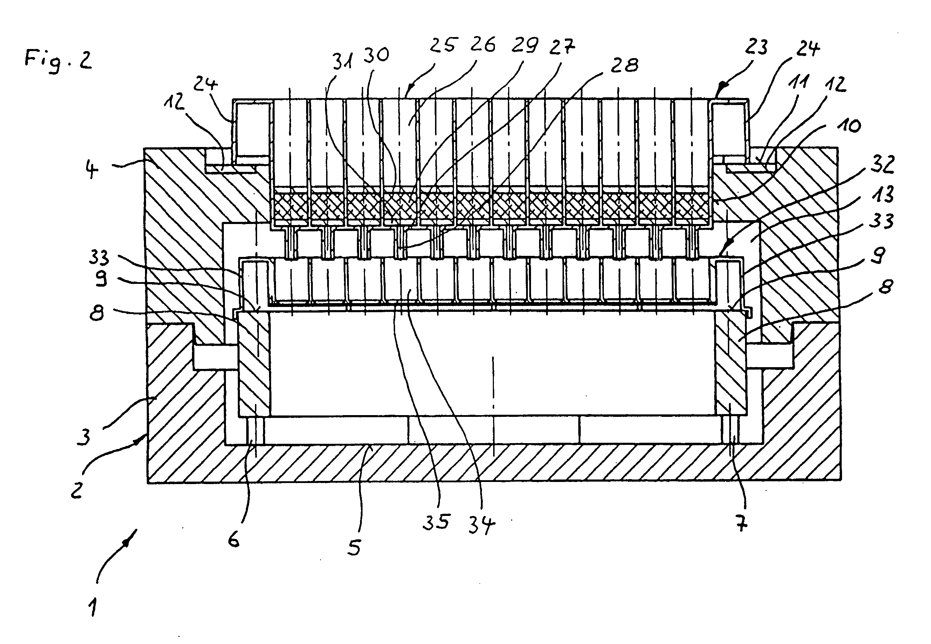

Nach dem Waschvorgang wird das Oberteil 4 von dem Unterteil 3 abgehoben. Sofern - wie bei der ersten Variante - die zweite Mikrotiterplatte 23 noch auf dem Abstützrahmen 8 ruht, wird diese entnommen. Der Abstützrahmen 8 ist dann frei, so daß nunmehr eine dritte Mikrotiterplatte 32 auf den Abstützrahmen 8 aufgesetzt werden kann. Diese Situation ist in Figur 2 dargestellt. Die dritte Mikrotiterplatte 32 weist gleichfalls einen umlaufenden Randsteg 33 auf, über den sie sich auf der Stützfläche 9 des Abstützrahmens 8 abstützt. Die dritte Mikrotiterplatte 32 weist 96 dritte Kavitäten - beispielhaft mit 34 bezeichnet - auf, die in dem gleichen 12x8-Raster angeordnet sind wie bei den ersten und zweiten Mikrotiterplatten 14, 25. Die dritten Kavitäten 34 werden untenseitig durch Ultrafiltrationsmembranen - beispielhaft mit 35 bezeichnet - abgeschlossen.

Nach dem Einsetzen der dritten Mikrotiterplatte 32 wird das Oberteil 4 wieder abdichtend auf das Unterteil 3 aufgesetzt und - soweit nicht schon bei der zweiten Variante vorher geschehen - die zweite Mikrotiterplatte 23 in die Öffnung 10 des Oberteils 4 eingesetzt, wobei sie sich mit ihrem Randsteg 24 auf dem Dichtring 12 abstützt. Die Auslauftüllen 28 der zweiten Mikrotiterplatte 23 ragen dann ein wenig in die dritten Kavitäten 34 hinein. Diese liegen jeweils koaxial zu den zweiten Kavitäten 25.

Danach wird mittels des Pipettierroboters ein Elutionsmittel, beispielsweise ein Elutionspuffer, in die zweiten Kavitäten 25 eingegeben. Der Elutionspuffer dringt in das Chromatographiematerial 29 ein und löst die dort adsorbtiv angelagerten Makromoleküle ab und schwemmt sie über die Auslauftüllen 28 in die dritten Kavitäten 34 ein. Sofern ein sehr dichtes Chromatographiematerial 29 verwendet wird, kann der Innenraum 13 zur Unterstützung des Durchflusses des Elutionspuffers erneut unter Vakuum gesetzt werden.

Nach Abschluß der Eluierung werden die zweite Mikrotiterplatte 23 aus der Öffnung 10 herausgenommen und das Oberteil 4 von dem Unterteil 3 abgehoben. Dann wird die dritte Mikrotiterplatte 32 entnommen und das Oberteil 4 wieder auf das Unterteil 3 aufgesetzt. Anschließend wird die dritte Mikrotiterplatte 32 in die Öffnung 10 des Oberteils 4 so eingesetzt, daß deren Randsteg 33 stirnseitig auf dem Dichtring 12 abdichtend aufsitzt.

Nunmehr wird der Innenraum 13 wieder mit Vakuum beaufschlagt. Das Vakuum sorgt dafür, daß der Elutionspuffer durch die Ultrafiltrationsmembranen 35 nach unten austritt und sich auf dem Boden 5 des Unterteils 3 sammelt. Als Retentat bleiben die zu gewinnenden Makromoleküle auf der Oberseite der Ultrafiltrationsmembranen 35 übrig. Zusätzlich kann in die dritten Kavitäten 34 ein Waschpuffer eingegeben werden, um eventuell noch vorhandene Salzreste auszuwaschen. Dieser Waschpuffer wird in gleicher Weise entfernt. Die zu gewinnenden Makromoleküle können schließlich nach Zugabe eines kleinen Volumens an Elutionspuffer mit Hilfe einer Pipette aus den dritten Kavitäten 34 entnommen werden. Dabei handelt es sich um hochreine Makromoleküle, die frei von partikulären Verunreinigungen sind und die den direkten Einsatz ohne weitere Aufkonzentrierungsschritte oder Nachreinigungsschritte für Folgeapplikationen, wie z.B. Transfektionsstudien, erlauben.

Im vorliegenden Beispiel sind die dritten Kavitäten 34 relativ kurz ausgebildet. Es versteht sich, daß sie nach unten hin verlängert ausgebildet sein können, um größere Elutionsvolumina aufnehmen zu können, wenn dies wegen der Menge der aufzureinigenden Makromoleküle und des daraus resultierenden Volumens des chromatographischen Materials 29 erforderlich ist. Alternativ dazu besteht die Möglichkeit, die Elution zunächst ohne Einsetzen der dritten Mikrotiterplatte 32 durchzuführen und das Elutionsvolumen in einer besonderen, auf dem Abstützrahmen 8 aufgesetzten, als Mikrotiterplatte ausgebildeten Auffangplatte mit unten geschlossenen, großvolumigen Kavitäten zu sammeln, aus dem Innenraum 13 zu entfernen und nach Austausch der zweiten Mikrotiterplatte 23 gegen die dritte Mikrotiterplatte 32 auf deren Kavitäten 34 portionsweise aufzugeben. Im nachfolgenden ist dieses Verfahren anhand eines praktischen Beispiels, nämlich der Aufreinigung eines Plasmides, näher erläutert:

### Lyse der Bakterien:

In einem entsprechenden Kulturkolben werden 100 ml LB-Medium mit Antibiotikum (Ampicillin Endkonzentration 100 µg/ml) versetzt und mit einer einzelnen Bakterienkolonie (z.B. E.coli Stamm DH5α, transformiert mit der Plasmid-DNA pcDNA3.1 HisB / lac Z (Invitrogen, Größe: 8.6 kbp) - von einer entsprechenden Agarplatte inoculiert. Die Anzucht der Bakterien erfolgt 12 - 14 Stunden bei 37°C in einem Schüttelinkubator. Nach der Anzucht der Bakterien werden je 1 ml der Bakterienkultur in je eine Vertiefung einer 96well Square-well Microplatte überführt. Alternativ kann die Anzucht der Bakterien auch direkt in den Square-well Platten durchgeführt werden, wobei sich die Anzuchtdauer auf 16-20 h verlängert. (Alle weiteren Volumenangaben beziehen sich auf jeweils eine Vertiefung der 96well Square-well Platte.) Nach dem Abzentrifugieren der Bakterienkultur werden die Pellets in 0,4 ml Resuspendierungspuffer (50 mM Tris/HCl pH 8.0, 10 mM EDTA, 100 µg RNase A / ml) resuspendiert, anschließend mit 0,4 ml Lysis Lösung (200 mM NaOH, 1% SDS) versetzt, vorsichtig gemischt und 5 min bei Raumtemperatur inkubiert. Anschließend werden 0,4 ml Neutralisierungspuffer (2.8 M Kaliumacetat, pH 5.1) versetzt, die Lösung durch Umschwenken durchmischt und 5 min auf Eis inkubiert.

Das Vakuumgerät 1 wird für die eigentliche Aufreinigung wie folgt vorbereitet:

In das wannenförmige Unterteil 3 des Vakuumgerätes 1 wird zunächst die mit einem Chromatographiematerial 29 befüllte zweite Mikrotiterplatte 23 mit Auflage auf dem Abstützrahmen 8 eingesetzt. In jede Kavität 25 dieser Mikrotiterplatte 23 wird 1 ml Äquilibierungspuffer N2-EF (100 mM Tris/Phosphat, pH 6.3, 15 % Ethanol, 900 mM KCl, 0.5% Triton X-114) eingefüllt. Das Vakuumgerät 1 wird mit dem Oberteil 4 verschlossen und in dieses die erste Mikrotiterplatte 14 zur nachfolgenden Klärung der Lysate eingesetzt.

### Aufreinigung:

Nachdem der Äquilibrierungspuffer die Mikrotiterplatte 23 durchströmt hat, wobei der Äquilibrierungspuffer die Mikrotiterplatte 23 allein durch Schwerkrafteinfluß durchströmt, werden die lysierten Bakterien in die Kavitäten 16 der Mikrotiterplatte 14 überführt. Zur Unterstützung der Filtration wird im Innenraum 13 des Vakuumgerätes 1 ein Vakuum erzeugt, wobei der Atmosphärendruck um 100 bis 150 mbar reduziert wird. In diesem Prozeß wird das Lysat gefiltert. Die löslichen Bestandteile treten aus der ersten Mikrotiterplatte 14 aus und in die zweite Mikrotiterplatte 23 ein. Dort läuft das gefilterte Lysat durch das Chromatographiematerial 29 der Mikrotiterplatte 23, wobei die zu reinigenden Plasmide am Chromatographiematerial 29 zurückgehalten werden.

Nach Abschluß dieser ersten Filtration wird die im Oberteil 4 befindliche erste Mikrotiterplatte 14 verworfen und die im Unterteil 3 befindliche zweite Mikrotiterplatte 23 in das Oberteil 4 eingesetzt. In das Unterteil 3 des Vakuumgerätes 1 wird zunächst keine Mikrotiterplatte eingesetzt.

Nun werden die folgenden Waschpuffer in die jetzt im Oberteil 4 befindliche Mikrotiterplatte 23, nacheinander in jede Kavität 25 aufgegeben: 2 x 1 ml Puffer N3-EF (100 mM Tris/Phosphat, pH 6.3, 15 % Ethanol, 1150 mM KCl, 0.5% Triton X-114), 2 x 1 ml N4-EF (100 mM Tris/Phosphat, pH 6.3, 15 % Ethanol, 1150 mM KC1). Auch hier können die Filtrationsschritte wie oben beschrieben durch Anlegen eines Vakuums unterstützt werden.

Nach Abschluß der Waschschritte wird in das Unterteil 3 des Vakuumgerätes 1 die Auffangplatte eingesetzt, die zum zwischenzeitlichen Auffangen der eluierten DNA dient. Nach Verschließen des Vakuumgerätes 1 werden in jede der Kavitäten 25 der sich im Oberteil 4 befindlichen Mikrotiterplatte 23 0.5 ml des Elutionspuffers N5-EF (100 mM Tris/Phosphat, pH 7.0, 15 % Ethanol, 1500 mM KCl) eingefüllt. Nachdem der Elutionspuffer die Kavitäten 25 der Mikrotiterplatte 23 vollständig passiert hat, wird diese verworfen.

### Entsalzung:

Die sich im Unterteil 3 des Vakuumgerätes 1 befindliche Auffangplatte, die jetzt die eluierte DNA enthält, wird aus dem Unterteil 3 entnommen. Das Vakuumgerät 1 wird verschlossen, und in das Oberteil 3 die zweite Mikrotiterplatte 23 gegen die dritte Mikrotiterplatte 32 zur Entsalzung durch Ultrafiltration der aufgereinigten DNA ausgetauscht. Die eluierte DNA wird dann aus der Auffangplatte in diese Mikrotiterplatte 32 übertragen. Zur Filtration wird erneut ein Vakuum angelegt, wobei der Druck im Innenraum 13 des Vakuumgerätes 1 um 400 mbar gegenüber dem Atmosphärendruck reduziert wird. Bei dieser Art der Entsalzung verbleiben die Nukleinsäuren in den Kavitäten der dritten Mikrotiterplatte 32. Nachdem die Eluate diese Mikrotiterplatte 32 vollständig passiert haben, wird in die einzelnen Vertiefungen nochmals 300 µl endotoxinfreier TE-Puffer zur weiteren Salzentfernung aufgetragen. Die Filtration wird durch ein Anlegen von Vakuum wie zuvor beschrieben unterstützt.

Nachdem die Waschlösung die Ultrafiltationsmembranen 35 passiert hat, wird der Innenraum des Vakuumgerätes 1 belüftet, die dritte Mikrotiterplatte 32 entnommen und die gereinigte und entsalzte DNA durch Zugabe von 50-100 µl endotoxinfreiem TE-Puffer oder Wasser in der dritten Mikrotiterplatte 32 rekonstituiert. Dazu kann diese Platte durch 5 min. Schütteln bei 250-400 Umdrehungen pro min auf einem geeigneten Schüttler plaziert werden. Alternativ kann die Rekonstitution der DNA durch ein 10 maliges auf- und abpipettieren unterstützt werden. Abschließend erfolgt das abpipettieren der gereinigten und entsalzent DNA aus der dritten Mikrotiterplatte 32 in eine geeignete Lagerungsplatte.

### Ergebnisse:

| Probe | µg DNA nach Entsalzung und Wiederaufnahme in 50 µl Puffer TE (Mittelwerte aus je 12 Präparationen |
|---|---|
| 1 | 10.85 |
| 2 | 10.8 |
| 3 | 11.6 |
| 4 | 10.4 |
| 5 | 11.15 |
| 6 | 9.85 |
| 7 | 13.65 |
| 8 | 9.35 |

Der Endotoxingehalt der Präparationen betrug <0.05 EU / µg DNA.

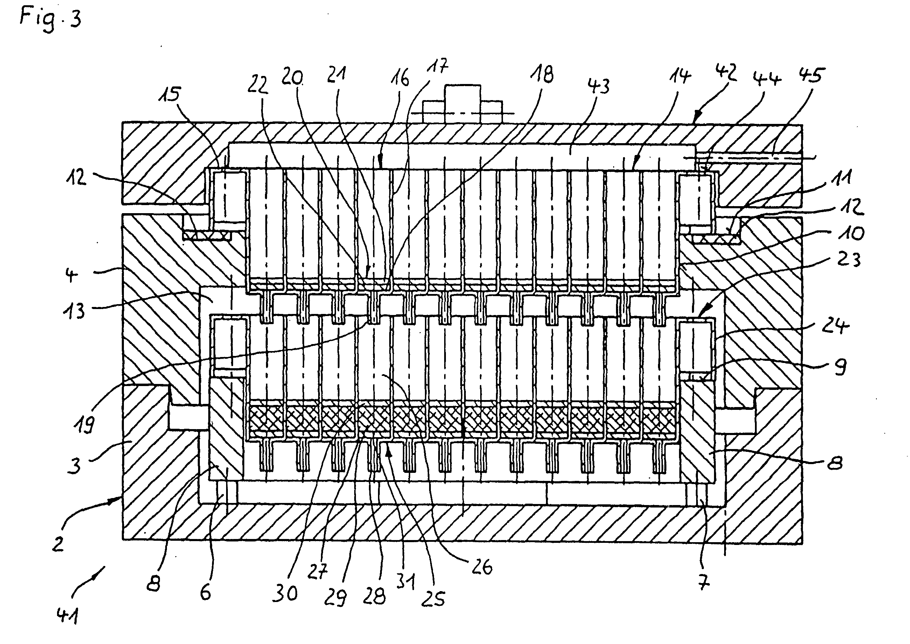

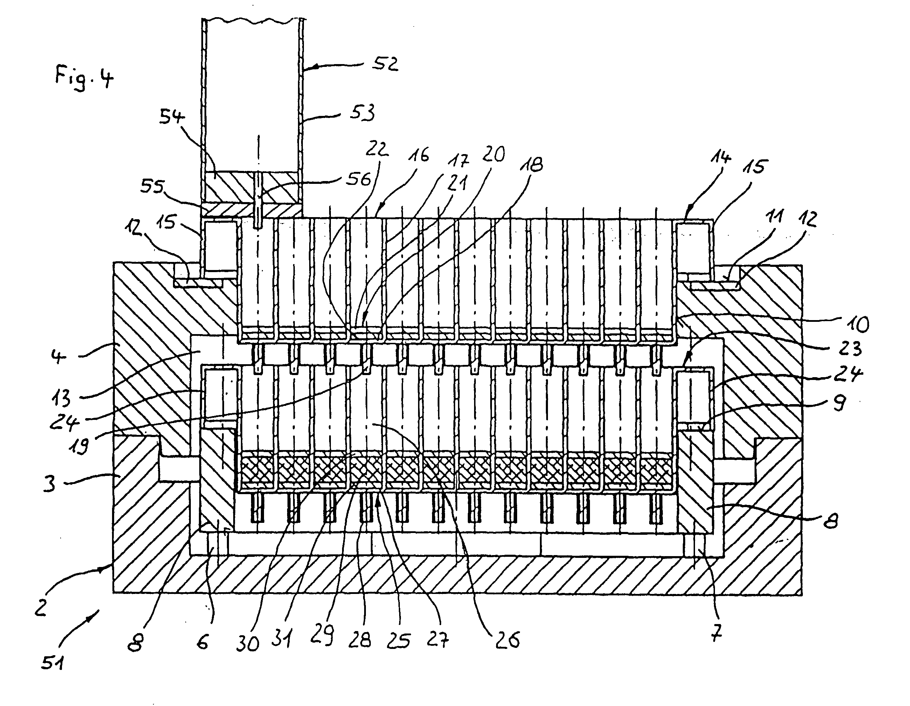

Die Figuren 3 und 4 zeigen ein Überdruckgerät 41, das bis auf eine Ausnahme mit dem Vakuumgerät gemäß Figur 1 identisch übereinstimmt. Deshalb sind in Figur 3 für gleiche Teile die gleichen Bezugsziffern verwendet worden wie in Figur 1. Bezüglich der mit den Bezugsziffern 1 bis 31 bezeichneten Teile wird auf die Beschreibung der Figur 1 Bezug genommen.

Das Überdruckgerät 41 weist - in Abweichung zu dem Vakuumgerät gemäß den Figuren 1 und 2 - zusätzlich einen Überdruckaufsatz 42 auf, der untenseitig eine Ausnehmung 43 aufweist, die von einem nach unten gerichteten Absatz 44 umgeben ist. Mit dem Absatz 44 liegt der Überdruckaufsatz 42 allseitig abdichtend auf der Oberseite des Randstegs 15 der ersten Mikrotiterplatte 14 auf und ist über hier nicht näher dargestellte Haltevorrichtungen mit dem Oberteil 4 verbunden. Über einen Kanal 45 in dem Überdruckaufsatz 42 kann die Ausnehmung 43 mit einer pneumatischen Überdruckquelle verbunden werden.

Das Überdruckgerät 41 wird im wesentlichen in der gleichen Weise gehandhabt wie das Vakuumgerät 1 gemäß den Figuren 1 und 2. Nach Abnahme des Überdruckaufsatzes 42 werden das zuvor gewonnene Lysat in die ersten Kavitäten 16 einpipettiert und dann der Überdruckaufsatz 42 wieder aufgesetzt. Danach werden die Teile des Überdruckgeräts 41 durch eine von außen wirkende äußere Druckkraft oder Haltevorrichtungen gasdicht miteinander verbunden. Die Ausnehmung 43 und damit die ersten Kavitäten 16 werden durch Anschalten der Überdruckquelle unter Druck gesetzt. Hierdurch wird das Lysat durch die Filtereinheiten 20 gedrückt und strömt dann über die Auslauftüllen 19 in die zweiten Kavitäten 25 der zweiten Mikrotiterplatte 23 als geklärtes Lysat ein.

Sofern das Chromatographiematerial 29 und die Fritten 30, 31 relativ durchlässig sind (erste Variante), durchströmt die Lösung das Chromatographiematerial 29 und die Fritten 30, 31 unter Adsorption der zu gewinnenden Makromoleküle am Chromatographiematerial 29. Danach wird der Überdruckaufsatz 42 zusammen mit der ersten Mikrotiterplatte 14 abgehoben, und es werden Waschpuffer in die Kavitäten 25 der zweiten Mikrotiterplatte 23 eingegeben. Sie dringen aufgrund Schwerkrafteinwirkung in das Chromatographiematerial 29 ein und unterstützen die Aufreinigung der zu gewinnenden Makromoleküle.

Sofern der Durchlauf der Lösung und des Waschpuffers durch das Chromatographiematerial 29 und die Fritten 30, 31 stark behindert oder sogar verhindert ist (zweite Variante), werden der Überdruckaufsatz 42 und die erste Mikrotiterplatte 14 nach Sammeln der Lösung in den zweiten Kavitäten 25 von dem Oberteil 4 abgenommen und das Oberteil 4 von dem Unterteil 3 abgehoben. Dann wird die zweite Mikrotiterplatte 23 entnommen, das Oberteil 4 wieder auf das Unterteil 3 aufgesetzt und die zweite Mikrotiterplatte 23 in die Öffnung 10 des Oberteils 4 eingesetzt. Anschließend wird der Überdruckaufsatz 42 auf die zweite Mikrotiterplatte 23 abdichtend aufgesetzt und die Ausnehmung 43 unter Überdruck gesetzt mit der Folge, daß die Lösung das Chromatographiematerial unter Adsorption der zu lösenden Makromoleküle durchläuft.

Nach dem Waschvorgang wird das Oberteil 4 mit dem Überdruckaufsatz 42 von dem Unterteil 3 abgehoben. Sofern - wie bei der ersten Variante - die zweite Mikrotiterplatte 23 noch auf dem Abstützrahmen 8 ruht, wird diese entnommen. Der Abstützrahmen ist dann frei, so daß nunmehr die dritte Mikrotiterplatte 32 an die Stelle der zweiten Mikrotiterplatte 23 auf den Abstützrahmen 8 aufgesetzt werden kann (vgl. Figur 2). Danach wird das Oberteil 4 wieder abdichtend auf das Unterteil 3 aufgesetzt und - soweit nicht wie schon bei der zweiten Variante vorher geschehen - die zweite Mikrotiterplatte 23 nach Abnahme des Überdruckaufsatzes 42 in die Öffnung 10 des Oberteils 4 eingesetzt. Anschließend wird der Elutionspuffer in die zweiten Kavitäten 25 eingegeben. Sollte der Elutionspuffer das Chromatographiematerial 29 nicht allein aufgrund Schwerkraft durchfließen können, wird der überdruckaufsatz 42 auf die zweite Mikrotiterplatte 23 aufgesetzt und die Ausnehmung 43 erneut mit Überdruck beaufschlagt. Auf diese Weise wird der Durchfluß des Elutionspuffers unterstützt.

Nach Abschluß der Eluierung werden - analog zu der Verfahrensweise bei dem Vakuumgerät gemäß den Figuren 1 und 2 - der Überdruckaufsatz 42 abgenommen und die zweite Mikrotiterplatte 23 aus der Öffnung 10 herausgenommen und das Oberteil 4 von dem Unterteil 3 abgehoben. Dann wird die dritte Mikrotiterplatte 32 entnommen und das Oberteil 4 wieder auf das Unterteil 3 aufgesetzt. Danach wird die dritte Mikrotiterplatte 32 in die Öffnung 10 des Oberteils 4 eingesetzt. Nach Aufsetzen des Überdruckaufsatzes 42 wird die Ausnehmung 43 wieder mit Überdruck beaufschlagt. Der Überdruck sorgt dafür, daß der Elutionspuffer durch die Ultrafiltrationsplatten 35 nach unten austritt, während als Retentat die zu gewinnenden Makromoleküle auf der Oberseite der Ultrafiltrationsplatten 35 übrigbleiben. Alles weitere geschieht so, wie zu dem Vakuumgerät 1 gemäß den Figuren 1 und 2 beschrieben.

Die in Figur 4 dargestellte Variante der Vorrichtung gemäß Figur 3 ist ebenfalls ein Überdruckgerät 51, das sich von dem Ausführungsbeispiel gemäß Figur 3 lediglich dadurch unterscheidet, daß statt des die erste Mikrotiterplatte 14 vollständig abdeckenden Überdruckaufsatzes 42 ein Überdruckaufsatz 52 verwendet wird, der in dem gezeigten Ausführungsbeispiel lediglich eine erste Kavität 16 abdeckt. Der Überdruckaufsatz 52 weist ein Zuführrohr 53 auf, das untenseitig durch einen Stopfen 54 verschlossen ist, dessen Außenseite mit einer Abdichtplatte 55 aus einem elastomeren Material versehen ist. Den Stopfen 54 und die Abdichtplatte 55 durchsetzt ein Austrittsröhrchen 56, das nach unten über die Abdichtplatte 55 vorsteht.

Es versteht sich, daß sich der Überdruckaufsatz 52 senkrecht zur Zeichnungsebene über mehrere oder alle der in dieser Richtung nebeneinander gereihten Kavitäten 16 erstrecken kann und dann für jede Kavität 16 ein Austrittsröhrchen 56 aufweist. Des weiteren besteht die Möglichkeit, den Überdruckaufsatz 52 analog in der Zeichnungsebene zu verbreitern bis hin dazu, daß der Überdruckaufsatz 52 sich über alle Kavitäten 16 erstreckt und für jede Kavität 16 ein Austrittsröhrchen 56 aufweist.

Der Überdruckaufsatz 51 wird von oben gegen die erste Mikrotiterplatte 14 unter Ausbildung einer gasdichten Verbindung gedrückt. Das Zuführrohr 53 ist an eine Überdruckquelle angeschlossen und kann - wie die Ausnehmung 43 des Überdruckaufsatzes 42 - mit Überdruck beaufschlagt werden. Der Überdruck pflanzt sich über das Austrittsröhrchen 56 in die darunterliegende Kavität 16 fort und treibt das zuvor einpipettierte Lysat durch die Filtereinheit 20, bis das Lysat als gereinigte Lösung vollständig in die darunterliegende Kavität 25 eingeflossen ist. Dann wird der Überdruckaufsatz 52 mit Hilfe des Pipettierroboters angehoben und entweder in der Zeichnungsebene oder senkrecht dazu um den Mittenabstand zweier benachbarter Kavitäten 16 versetzt und wieder so auf die benachbarte Kavität 16 abgesetzt, daß die Abdichtplatte 55 abdichtend wirkt.

Bis auf die Tatsache, daß die einzelnen Kavitäten 16 im Unterschied zu dem Überdruckgerät 41 gemäß Figur 3 nicht gleichzeitig, sondern nacheinander mit Überdruck beaufschlagt werden, unterscheidet sich die Verfahrensweise nicht von derjenigen, wie zu dem Überdruckgerät 41 gemäß Figur 3 beschrieben, so daß auf die dortige Beschreibung Bezug genommen wird.

## Patentansprüche

1. Verfahren zur Isolierung biologischer Makromoleküle, insbesondere von Plasmid-DNA aus biologischem Ausgangsmaterial, bei dem aus dem Ausgangsmaterial durch Aufschluß ein Lysat gebildet, dies gereinigt und die so gewonnene Lösung auf ein chromatographisches Material (29) gegeben wird, das die Makromoleküle adsorbiert, wobei zuvor oder gleichzeitig eine Abreicherung von Endotoxinen durchgeführt wird, und bei dem die aufgereinigten Makromoleküle mit Hilfe eines Elutionsmittels von dem chromatographischen Material (29) eluiert und schließlich von dem Elutionsmittel befreit werden, **gekennzeichnet durch** folgende Verfahrensschritte:
(1) in einer Vorrichtung (1, 41, 51) werden eine erste Mikrotiterplatte (14) mit mehreren parallelen ersten Kavitäten (16) und eine zweite Mikrotiterplatte (23) mit mehreren zweiten Kavitäten (25) derart angeordnet, daß jeweils eine erste und eine zweite Kavität (16, 25) hintereinanderliegen;
(2) in den ersten Kavitäten (16) wurden Filtereinheiten (20) angeordnet;
(3) in den zweiten Kavitäten (25) wurde das chromatographische Material (29) angeordnet;
(4) in die ersten Kavitäten (16) wird das Lysat eingegeben;
(5) die aus den ersten Kavitäten (16) austretende Lösung wird direkt in die zweiten Kavitäten (25) überführt, und die Makromoleküle werden an dem chromatographischen Material (29) adsorbiert;
(6) die Endotoxine und weitere Verunreinigungen werden mit einem Waschmittel ausgewaschen;
(7) in die zweiten Kavitäten (25) wird das Elutionsmittel aufgegeben, so daß eine Mischung aus Elutionsmittel und Makromolekülen aus der zweiten Mikrotiterplatte (23) austritt;
(8) die Mischung wird in eine dritte Mikrotiterplatte (32) mit mehreren parallelen dritten Kavitäten (34) überführt;
(9) in den dritten Kavitäten (34) wurden Ultrafiltrationseinheiten (35) angeordnet;
(10) die dritten Kavitäten (34) werden einer in Richtung deren Austrittsseite Kraft ausübenden Druckdifferenz und/oder Fliehkraft ausgesetzt;
(11) danach werden die Makromoleküle als Retentat gewonnen.

2. Verfahren nach Anspruch 1, **dadurch gekennzeichnet, daß** das chromatographische Material (29) vor der Aufgabe der Lösung mit einem Puffer äquilibriert wird.

3. Verfahren nach Anspruch 1 oder 2, **dadurch gekennzeichnet, daß** nach Aufgabe des Lysats in die ersten Kavitäten (16) diesen eine in Richtung auf die zweiten Kavitäten (25) Kraft ausübende Druckdifferenz und/oder Fliehkraft aufgeprägt wird.

4. Verfahren nach einem der Ansprüche 1 bis 3, **dadurch gekennzeichnet, daß** die zweiten Kavitäten (25) für die Unterstützung des Durchflusses der Lösung und/oder des Elutionsmittels einer in Richtung deren Austrittsseite Kraft ausübenden Druckdifferenz und/oder Fliehkraft ausgesetzt werden.

5. Verfahren nach einem der Ansprüche 1 bis 4, **dadurch gekennzeichnet, daß** nach Überführung der Lösung in die zweiten Kavitäten (25) die erste Mikrotiterplatte (14) gegen die zweite Mikrotiterplatte (23) ausgetauscht wird.

6. Verfahren nach einem der Ansprüche 1 bis 5, **dadurch gekennzeichnet, daß** zur Elution der Makromoleküle die dritte Mikrotiterplatte (32) so in Bezug zu der zweiten Mikrotiterplatte (23) angeordnet wird, daß jeweils eine zweite und eine dritte Kavität (25, 34) axial hintereinander liegen.

7. Verfahren nach Anspruch 5 und 6, **dadurch gekennzeichnet, daß** zur Trennung des Elutionsmittels von den Makromolekülen die dritte Mikrotiterplatte (32) in die vorherige Position der zweiten Mikrotiterplatte (23) gebracht wird.

8. Verfahren nach Anspruch 5, **dadurch gekennzeichnet, daß** zur Elution der Makromoleküle an die freie Stelle, die zuvor von der zweiten Mikrotiterplatte (23) eingenommen worden ist, eine Auffangplatte zum Auffangen der Mischung aus Elutionsmittel und Makromoleküle angeordnet wird, daß nach der Elution der Makromoleküle die zweite Mikrotiterplatte (23) durch die dritte Mikrotiterplatte (32) ersetzt wird und die Mischung aus Elutionsmittel und Makromolekülen von der Auffangplatte in die dritten Kavitäten (34) überführt wird.

9. Verfahren nach einem der Ansprüche 1 bis 8, **dadurch gekennzeichnet, daß** das Waschmittel ein nicht ionisches Tensid enthält.

10. Verfahren nach einem der Ansprüche 1 bis 9, **dadurch gekennzeichnet, daß** das Waschmittel an Einsätze (30, 31) gebunden ist, die in die zweiten Kavitäten (25) eingebracht wurden.

11. Verfahren nach Anspruch 10, **dadurch gekennzeichnet, daß** als Einsätze Fritten (30, 31) verwendet werden, die mit dem Waschmittel imprägniert sind.

12. Verfahren nach einem der Ansprüche 9 bis 11, **dadurch gekennzeichnet, daß** das chromatographische Material mit dem Waschmittel imprägniert wird.

13. Verfahren nach einem der Ansprüche 1 bis 12, **dadurch gekennzeichnet, daß** als chromatographisches Material Anionenaustauschermaterial (29) verwendet wird.

14. Verfahren nach einem der Ansprüche 1 bis 13, **dadurch gekennzeichnet, daß** das chromatographische Material (29) vor der Eluierung der Makromoleküle mehrfach gewaschen wird.

15. Verfahren nach einem der Ansprüche 1 bis 14, **dadurch gekennzeichnet, daß** die Filtereinheiten (20) mehrstufig wirken.

16. Verfahren nach einem der Ansprüche 1 bis 15, **dadurch gekennzeichnet, daß** für die Trennung des Elutionsmittels von den Makromolekülen mehrere dritte Mikrotiterplatten (32) mit Ultrafiltrationseinheiten (35) verwendet werden.

17. Vorrichtung zur Durchführung des Verfahrens nach einem der Ansprüche 1 bis 16, **gekennzeichnet durch** folgende Merkmale:
(12) die Vorrichtung (1, 41, 51) hat ein Gehäuse (2);
(13) die Vorrichtung (1, 41, 51) weist erste, zweite und dritte Mikrotiterplatten (14, 23, 32) mit jeweils mehreren ersten, zweiten bzw. dritten Kavitäten (16, 25, 34) in einem übereinstimmenden Raster auf;
(14) in den ersten Kavitäten (16) sind Filtereinheiten (20) angeordnet;
(15) in den zweiten Kavitäten (25) in chromatographisches Material (29) angeordnet;
(16) in den dritten Kavitäten (34) sind Ultrafiltrationseinheiten (35) angeordnet;
(17) die Vorrichtung (1, 41, 51) weist eine erste Stützfläche (11) für eine randseitig abdichtende Auflage der Mikrotiterplatten (14, 23, 32) auf;
(18) die Vorrichtung (1, 41, 51) weist eine zweite Stützfläche (9) für die Mikrotiterplatten (14, 23, 32) auf;
(19) die Stützflächen (9, 11) sind so angeordnet, daß die Kavitäten (16, 25, 34) von zwei darauf aufgelegten Mikrotiterplatten (14, 23, 32) jeweils axial hintereinanderliegen;
(20) der auf der ersten Stützfläche (11) aufliegenden Mikrotiterplatte (14, 23, 32) ist ein Differenzdruck aufprägbar, der in zumindest einer Kavität (16, 25, 34) dieser Mikrotiterplatte (14, 23, 32) eine in Richtung auf die zweite Stützfläche (9) wirkende Kraft ausübt.

18. Vorrichtung nach Anspruch 17, **dadurch gekennzeichnet, daß** das Gehäuse (2) ein Unterteil (3) mit Auffangwanne (5) und ein darauf abnehmbar aufgesetztes Oberteil (4) aufweist und daß die zweite Stützfläche (9) am Unterteil (3) so angeordnet ist, daß eine darauf aufliegende Mikrotiterplatte (23, 32) nach Entfernen des Oberteils (4) herausnehmbar ist.

19. Vorrichtung nach Anspruch 18, **dadurch gekennzeichnet, daß** die erste Stützfläche (11) am Oberteil (4) so angeordnet ist, daß die darauf angeordnete erste Mikrotiterplatte (14, 23, 32) gegen eine andere Mikrotiterplatte (14, 23, 32) und diese gegen die dritte Mikrotiterplatte austauschbar sind.

20. Vorrichtung nach Anspruch 18 oder 19, **dadurch gekennzeichnet, daß** der von Ober- und Unterteil (3, 4) umschlossene Raum (13) mit einer Unterdruckquelle verbunden ist.

21. Vorrichtung nach einem der Ansprüche 17 bis 20, **dadurch gekennzeichnet, daß** ein Raum (43, 53) mit zumindest einer Kavität (16, 25, 34) der auf der ersten Stützfläche (11) ruhenden Mikrotiterplatte (14, 23, 32) auf der Seite dieser Mikrotiterplatte (14, 23, 32) verbunden ist, der der zweiten Stützfläche (9) abgewandt ist, und daß dieser Raum (43, 53) an eine Überdruckquelle angeschlossen ist.

22. Vorrichtung nach einem der Ansprüche 17 bis 21, **dadurch gekennzeichnet, daß** die Filtereinheiten (20) in der ersten Mikrotiterplatte (14) wenigstens einen Grob- und einen Feinfilter (21, 22) aufweisen.

23. Vorrichtung nach einem der Ansprüche 17 bis 22, **dadurch gekennzeichnet, daß** das chromatographische Material ein Anionenaustauschermaterial (29) ist.

24. Vorrichtung nach einem der Ansprüche 17 bis 23, **dadurch gekennzeichnet, daß** das chromatographische Material (29) zwischen zwei Fritten (30, 31) angeordnet ist oder als monolithischer Block oder als Membran vorliegt.

25. Vorrichtung nach Anspruch 24, **dadurch gekennzeichnet, daß** die Fritten (30, 31) mit einem die Abreicherung von Endotoxinen bewirkenden Waschmittel versehen sind.

26. Vorrichtung nach einem der Ansprüche 17 bis 25, **dadurch gekennzeichnet, daß** das chromatographische Material (29) mit einem die Abreicherung von Endotoxinen bewirkenden Waschmittel versehen ist.

## Claims

1. Method of isolating biological macromolecules, in particular plasmid DNA, from biological starting material, in which a lysate is formed from the starting material by dissolution and is purified, and the solution thus obtained is placed on chromatographic material (29) which adsorbs the macromolecules, a depletion of endotoxins being effected beforehand or simultaneously, and in which the purified macromolecules are eluted from the chromatographic material (29) using an elution agent and finally are freed of the elution agent, **characterized by** the following procedural steps:
(1) a first microtiter plate (14) having a plurality of parallel first cavities (16) and a second microtiter plate (23) having a plurality of second cavities (25) are disposed in an apparatus (1, 41, 51) in such a way that respectively a first and a second cavity (16, 25) lie one behind the other;
(2) filter units (20) were disposed in the first cavities (16);
(3) the chromatographic material (29) was disposed in the second cavities (25);
(4) the lysate is introduced into the first cavities (16);
(5) the solution emerging from the first cavities (16) is conveyed directly into the second cavities (25) and the macromolecules are adsorbed on the chromatographic material (29);
(6) the endotoxins and additional impurities are washed out using a washing agent;
(7) the elution agent is fed into the second cavities (25), so that a mixture of elution agent and macromolecules emerges from the second microtiter plate (23);
(8) the mixture is conveyed into a third microtiter plate (32) having a plurality of parallel third cavities (34);
(9) ultrafiltration units (35) were disposed in the third cavities (34);
(10) the third cavities (34) are subjected to a pressure difference and/or centrifugal force which exerts a force in the direction of their outlet side;
(11) the macromolecules are then obtained as a retentate.

2. Method according to Claim 1, **characterized in that,** prior to feeding in of the solution, the chromatographic material (29) is equilibrated with a buffer.

3. Method according to Claim 1 or 2, **characterized in that,** after feeding of the lysate into the first cavities (16), these are subjected to a pressure difference and/or centrifugal force which exerts a force in direction of the second cavities (25).

4. Method according to one of Claims 1 to 3, **characterized in that**, for the purpose of encouraging the flow of solution and/or elution agent, the second cavities (25) are subjected to a pressure difference and/or centrifugal force which exerts a force in direction of their outlet side.

5. Method according to one of Claims 1 to 4, **characterized in that**, after the solution has been conveyed into the second cavities (25), the first microtiter plate (14) is exchanged for the second microtiter plate (23).

6. Method according to one of Claims 1 to 5, **characterized in that**, for elution of the macromolecules, the third microtiter plate (32) is disposed relative to the second microtiter plate (23) in such a way that respectively a second and a third cavity (25, 34) lie one behind the other in axial direction.

7. Method according to Claims 5 and 6, **characterized in that**, for separation of the elution agent from the macromolecules, the third microtiter plate (32) is placed in the position previously occupied by the second microtiter plate (23).

8. Method according to Claim 5, **characterized in that**, for elution of the macromolecules, a collecting plate is disposed in the free space previously occupied by the second microtiter plate (23) for collecting the mixture of elution agent and macromolecules, and **in that**, after elution of the macromolecules, the second microtiter plate (23) is replaced by the third microtiter plate (32) and the mixture of elution agent and macromolecules is conveyed from the collecting plate into the third cavities (34).

9. Method according to one of Claims 1 to 8, **characterized in that** the washing agent contains a non-ionic surfactant.

10. Method according to one of Claims 1 to 9, **characterized in that** the washing agent is incorporated into inserts (30, 31), which were placed in the second cavities (25).

11. Method according to Claim 10, **characterized in that** frits (30, 31) are used as inserts and are impregnated with the washing agent.

12. Method according to one of Claims 9 to 11, **characterized in that** the chromatographic material is impregnated with the washing agent.

13. Method according to one of Claims 1 to 12, **characterized in that** anion exchanger material (29) is used as chromatographic material.

14. Method according to one of Claims 1 to 13, **characterized in that** the chromatographic material (29) is washed repeatedly prior to elution of the macromolecules.

15. Method according to one of Claims 1 to 14, **characterized in that** the filter units (20) work in several stages.

16. Method according to one of Claims 1 to 15, **characterized in that,** for separation of the elution agent from the macromolecules, a plurality of third microtiter plates (32) with ultrafiltration units (35) are used.

17. Apparatus for implementing the method according to one of Claims 1 to 16, **characterized by** the following features:
(12) the apparatus (1, 41, 51) has a housing (2);
(13) the apparatus (1, 41, 51) has first, second and third microtiter plates (14, 23, 32), having a plurality of first, second and third cavities (16, 25, 34) respectively, in matching rasters;
(14) filter units (20) are disposed in the first cavities (16);
(15) chromatographic material (29) is disposed in the second cavities (25);
(16) ultrafiltration units (35) are disposed in the third cavities (34);
(17) the apparatus (1, 41, 51) has a first surface (11) with peripheral sealing means for supporting the microtiter plates (14, 23, 32);
(18) the apparatus (1, 41, 51) has a second surface (9) for supporting the microtiter plates (14, 23, 32);
(19) the supporting surfaces (9, 11) are disposed in such a way that the cavities (16, 25, 34) of two microtiter plates (14, 23, 32) placed thereon are respectively located one behind the other in axial direction;
(20) the microtiter plate (14, 23, 32) resting on the first supporting surface (11) can be subjected to a differential pressure, which exerts, at least in one cavity (16, 25, 34) of this microtiter plate (14, 23, 32), a force acting in direction of the second supporting surface (9).

18. Apparatus according to Claim 17, **characterized in that** the housing (2) comprises a lower portion (3) together with collecting trough (5), and an upper portion (4) mounted thereon which is detachable, and **in that** the second supporting surface (9) on the lower portion (3) is disposed in such a way that a microtiter plate (23, 32) resting thereon can be taken out after the upper portion (4) has been removed.

19. Apparatus according to Claim 18, **characterized in that** the first supporting surface (11) is disposed on the upper portion (4) in such a way that the first microtiter plate (14, 23, 32) disposed thereon can be exchanged for another microtiter plate (14, 23, 32) and this can be exchanged for the third microtiter plate.

20. Apparatus according to Claim 18 or 19, **characterized in that** the space (13) enclosed by the upper and lower portions (3, 4) is connected to a vacuum source.

21. Apparatus according to one of Claims 17 to 20, **characterized in that** a space (43, 53), disposed on the side of the microtiter plate (14, 23, 32) facing away from the second supporting surface (9), communicates with at least one cavity (16, 25, 34) of the microtiter plate (14, 23, 32) resting on the first supporting surface (11), **and in that** this space (43, 53) is attached to a pressure source.

22. Apparatus according to one of Claims 17 to 21, **characterized in that** the filter units (20) in the first microtiter plate (14) include at least one coarse and one fme filter (21, 22).

23. Apparatus according to one of Claims 17 to 22, **characterized in that** the chromatographic material is an anion exchanger material (29).

24. Apparatus according to one of Claims 17 to 23, **characterized in that** the chromatographic material (29) is disposed between two frits (30, 31) or else it takes the form of a monolithic block or a membrane.

25. Apparatus according to Claim 24, **characterized in that** the frits (30, 31) are provided with a washing agent leading to the depletion of endotoxins.

26. Apparatus according to one of Claims 17 to 25, **characterized in that** the chromatographic material (29) is provided with a washing agent leading to the depletion of endotoxins.

## Revendications

1. Procédé d'isolation de macromolécules biologiques, en particulier d'ADN de plasmide issu d'un matériau de départ biologique, dans lequel on forme un lysat à partir d'un matériau de départ par dissolution, on le purifie et on place la solution ainsi obtenue sur un matériau chromatographique (29) qui adsorbe les macromolécules, en réalisant avant ou simultanément, un appauvrissement d'endotoxines, et dans lequel les macromolécules purifiées sont éluées du matériau chromatographique (29 ) à l'aide d'un agent d'élution et enfin libérées de l'agent d'élution, **caractérisé par** les étapes de procédé suivantes :
(1) dans un dispositif (1, 41, 51), on dispose une première plaque de microtitration (14) comportant plusieurs premières cavités parallèles (16) et une seconde plaque de microtitration (23) comportant plusieurs secondes cavités (25) agencées de façon telle qu'une première et une seconde cavités (16, 25) sont respectivement placées l'une derrière l'autre ;
(2) dans les premières cavités (16), on a disposé des unités de filtration (20) ;
(3) dans les secondes cavités (25), on a placé le matériau chromatographique (29) ;
(4) dans les premières cavités (16), on introduit le lysat ;
(5) la solution sortant des premières cavités (16) est directement transférée dans les secondes cavités (25), et les macromolécules sont adsorbées au niveau du matériau chromatographique (29) ;
(6) les endotoxines et d'autres impuretés sont enlevées par lavage avec un agent de lavage ;
(7) dans les secondes cavités (25), on charge l'agent d'élution, de sorte qu'un mélange composé de l'agent d'élution et des macromolécules sort de la seconde plaque de microtitration (23) ;
(8) le mélange est transféré dans une troisième plaque de microtitration (32) comportant plusieurs troisièmes cavités parallèles (34) ;
(9) dans les troisièmes cavités (34), on a disposé des unités d'ultrafiltration (35) ;
(10) les troisièmes cavités (34) sont soumises à une différence de pression et/ou à une force centrifuge exerçant une force dans la direction du côté de leur sortie ;
(11) les macromolécules sont ensuite recueillies sous la forme de rétentat.

2. Procédé selon la revendication 1, **caractérisé en ce que** le matériau chromatographique (29) est équilibré avec un tampon avant la charge de la solution.

3. Procédé selon la revendication 1 ou 2, **caractérisé en ce que**, après la charge du lysat dans les premières cavités (16), on imprime sur celles-ci une différence de pression et/ou une force centrifuge exerçant une force dans la direction des secondes cavités (25).

4. Procédé selon l'une des revendications 1 à 3, **caractérisé en ce que** les secondes cavités (25) sont soumises, pour assister la circulation de la solution et/ou de l'agent d'élution, à une différence de pression et/ou à une force centrifuge exerçant une force dans la direction du côté de leur sortie.

5. Procédé selon l'une des revendications 1 à 4, **caractérisé en ce qu'**après transfert de la solution dans les secondes cavités (25), on échange la première plaque de microtitration (14) avec la seconde plaque de microtitration (23).

6. Procédé selon l'une des revendications 1 à 5, **caractérisé en ce que**, pour l'élution des macromolécules, la troisième plaque de microtitration (32) est disposée par rapport à la seconde plaque de microtitration (23) de façon telle qu'une seconde et une troisième cavités (25, 34) sont disposés respectivement axialement l'une derrière l'autre.

7. Procédé selon la revendication 5 et 6, **caractérisé en ce que**, pour séparer l'agent d'élution des macromolécules, on amène la troisième plaque de microtitration (32) dans la position précédente de la seconde plaque de microtitration (23).

8. Procédé selon la revendication 5, **caractérisé en ce que**, pour l'élution des macromolécules à la position libre qui a été précédemment occupée par la seconde plaque de microtitration (23), on dispose une plaque collectrice destinée à capter ou recevoir le mélange composé de l'agent d'élution et des macromolécules de façon telle qu'après l'élution des macromolécules, la seconde plaque de microtitration (23) est remplacée par la troisième plaque de microtitration (32) et le mélange composé de l'agent d'élution et des macromolécules est transféré de la plaque collectrice dans les troisièmes cavités (34).

9. Procédé selon l'une des revendications 1 à 8, **caractérisé en ce que** l'agent de lavage contient un tensioactif non ionique.

10. Procédé selon l'une des revendications 1 à 9, **caractérisé en ce que** l'agent de lavage est lié à des garnitures (30, 31) qui ont été introduites dans les secondes cavités (25).

11. Procédé selon la revendication 10, **caractérisé en ce que** l'on utilise en tant que garnitures des frittes (30, 31) qui sont imprégnées de l'agent de lavage.

12. Procédé selon l'une des revendications 9 à 11, **caractérisé en ce que** le matériau chromatographique est imprégné de l'agent de lavage.

13. Procédé selon l'une des revendications 1 à 12, **caractérisé en ce que** l'on utilise comme matériau chromatographique un matériau échangeur d'anions (29).

14. Procédé selon l'une des revendications 1 à 13, **caractérisé en ce que** le matériau chromatographique (29) est lavé plusieurs fois avant l'élution des macromolécules.

15. Procédé selon l'une des revendications 1 à 14, **caractérisé en ce que** les unités de filtration (20) agissent en plusieurs étapes.

16. Procédé selon l'une des revendications 1 à 15, **caractérisé en ce que** pour la séparation de l'agent d'élution des macromolécules, on utilise plusieurs troisièmes plaques de microtitration (32) comportant des unités d'ultrafiltration (35).

17. Dispositif de réalisation du procédé selon l'une des revendications 1 à 16, **caractérisé par** les caractéristiques suivantes :
(12) le dispositif (1, 41, 51) comporte un boîtier (2) ;
(13) le dispositif (1, 41, 51) comporte des première, seconde et troisième plaques de microtitration (14, 23, 32) comportant respectivement plusieurs premières, secondes ou troisièmes cavités (16, 25, 34) en un réseau correspondant ;
(14) des unités de filtration (20) sont disposées dans les premières cavités (16) ;
(15) un matériau chromatographique (29) est disposé dans les secondes cavités (25) ;
(16) des unités d'ultrafiltration (35) sont disposées dans les troisièmes cavités (34) ;
(17) le dispositif (1, 41, 51) comporte une première surface d'appui (11) pour un placement des plaques de microtitration (14, 23, 32) permettant une étanchéification au niveau du bord ;
(18) le dispositif (1, 41, 51) comporte une seconde surface d'appui (9) pour les plaques de microtitration (14, 23, 32) ;
(19) les surfaces d'appui (9, 11) sont disposées de façon telle que les cavités (16, 25, 34) de deux plaques de microtitration qui y sont placées dessus (14, 23, 32) sont respectivement disposés l'une derrière l'autre dans la direction axiale ;
(20) on peut imprimer sur la plaque de microtitration (14, 23, 32) reposant sur la première surface d'appui (11) une pression différentielle qui exerce dans au moins une cavité (16, 25, 34) de cette plaque de microtitration (14, 23, 32) une force qui agit dans la direction de la seconde surface d'appui (9).

18. Dispositif selon la revendication 17, **caractérisé en ce que** le boîtier (2) présente une partie inférieure (3) comportant une cuvette collectrice (5) et une partie supérieure qui y est placée dessus de façon amovible (4), et **en ce que** la seconde surface d'appui (9) est disposée dans la partie inférieure (3) de façon telle que l'on peut retirer une plaque de microtitration (23, 32) qui y est placée dessus après enlèvement de la partie supérieure (4).

19. Dispositif selon la revendication 18, **caractérisé en ce que** la première surface d'appui (11) est disposée sur ou au niveau de la partie supérieure (4) de façon telle que la première plaque de microtitration (14, 23, 32) qui y est placée dessus peut être échangée contre une autre plaque de microtitration (14, 23, 32) et celle-ci peut être échangée contre la troisième plaque de microtitration.

20. Dispositif selon la revendication 18 ou 19, **caractérisé en ce que** l'espace (13) compris entre la partie supérieure et la partie inférieure (3, 4) est relié à une source de dépression.

21. Dispositif selon l'une des revendications 17 à 20, **caractérisé en ce qu'**un espace (43, 53) est relié à au moins une cavité (16, 25, 34) de la plaque de microtitration (14, 23, 32) qui repose sur la première surface d'appui (11), sur le côté de cette plaque de microtitration (14, 23, 32), qui est éloigné de la seconde surface d'appui (9), et **en ce que** cet espace (43, 53) est raccordé à une source de surpression.

22. Dispositif selon l'une des revendications 17 à 21, **caractérisé en ce que** les unités de filtration (20) dans la première plaque de microfiltration (14) présentent au moins un filtre grossier et un filtre fin (21, 22).

23. Dispositif selon l'une des revendications 17 à 22, **caractérisé en ce que** le matériau chromatographique est un matériau échangeur d'anions (29).

24. Dispositif selon l'une des revendications 17 à 23, **caractérisé en ce que** le matériau chromatographique (29) est disposé entre deux frittes (30, 31) ou se présente sous la forme d'un bloc monolithique ou d'une membrane.

25. Dispositif selon la revendication 24, **caractérisé en ce que** les frittes (30, 31) sont pourvues d'un agent de lavage entraînant l'appauvrissement en endotoxines.

26. Dispositif selon l'une des revendications 17 à 25, **caractérisé en ce que** le matériau chromatographique (29) est pourvu d'un agent de lavage entraînant l'appauvrissement en endotoxines.
